# EUROPEAN PATENT APPLICATION

(11) **EP 4 811 003 A1**
(43) Date of publication of application: **23.09.2026**
(21) Application number: 26166159.9
(22) Date of filing: 19.03.2026
(51) Int. Cl.: G01T 1/17, G01T 1/29, A61B 6/03

(54) **POSITRON EMISSION TOMOGRAPHY SYSTEMS**

(30) Priority: 19.03.2025 CN 202510331094
(71) Applicant: Shanghai United Imaging Healthcare Co., Ltd., Shanghai 201807 (CN)
(72) Inventor: SUN, Yixing, Shanghai, 201807 (CN); CHU, Shaoping, Shanghai, 201807 (CN); LIU, Yilin, Shanghai, 201807 (CN)
(74) Representative: Wang, Bo

(57) **Abstract**

Embodiments of the present disclosure provide a PET system. A PET system comprises multiple detector rings, a first processing card and a second processing card. The multiple detector rings are arranged side by side along an axial direction of the PET system, each of the multiple detector rings includes a plurality of detector modules arranged along a circumferential direction of the detector ring. The first processing card is communicatively connected to the multiple detector rings, wherein the first processing card is configured to receive detection data with a same circumferential index, and determine a target event based on the detection data. The second processing card is communicatively connected to the first processing card, and is configured to determine a coincidence event pair by processing the target event.

## Description

### CROSS-REFERENCE TO RELATED APPLICATIONS

This application claims priority to Chinese Patent Application No. 202510331094.9, filed on March 19, 2025, the entire contents of which are hereby incorporated herein by reference.

### TECHNICAL FIELD

The present disclosure relates to the field of imaging technology, and in particular, to Positron Emission Tomography (PET) systems.

### BACKGROUND

PET imaging technology can non-invasively reveal metabolic, functional, and pathological states in a living organism, and has an irreplaceable value in fields such as early diagnosis of diseases, efficacy evaluation, and drug development. With a greatly expanded axial field of view, a long-axial PET imaging device achieves a technological leap from Localized static imaging to Whole-body multi-organ synchronous dynamic imaging, representing one of key directions in the development of molecular imaging. However, a count of detector rings in the long-axial PET imaging device has increased significantly, resulting in a tremendous amount of detection data being received per unit time, which poses a severe challenge to a data processing capability of a back-end electronic system, especially in terms of computational resources and data transmission bandwidth required for screening coincidence event pairs from massive data (i.e., a coincidence determination process).

Therefore, it is necessary to provide a PET system to reduce backend computing resource overhead and improve the efficiency, reliability, and stability of data processing.

### SUMMARY

One or more embodiments of the present disclosure provide a PET system. The PET system comprises multiple detector rings, a first processing card and a second processing card. The multiple detector rings are arranged side by side along an axial direction of the PET system, each of the multiple detector rings includes a plurality of detector modules arranged along a circumferential direction of the detector ring. The first processing card is communicatively connected to the multiple detector rings, wherein the first processing card is configured to receive detection data with a same circumferential index, and determine a target event based on the detection data. The second processing card is communicatively connected to the first processing card, and is configured to determine a coincidence event pair by processing the target event.

In some embodiments, the first processing card is further configured to determine the target event based on a target single event within a same time granularity in the detection data.

In some embodiments, in response to the target single event including one single event, the first processing card is further configured to designate the single event as the target event; and in response to the target single event including at least two single events, the first processing card is further configured to select one from the at least two single events as the target event.

In some embodiments, the second processing card is further configured to determine a candidate coincidence event based on the target event; determine a target event that meets a preset condition from target events, the target events are within a preset time window and correspond to target detector modules, the target detector modules are positioned opposite or approximately opposite to the detector module corresponding to the candidate coincidence event; designate the candidate coincidence event and the target event that meets the preset condition as a candidate coincidence event pair; and determine the coincidence event pair based on the candidate coincidence event pair.

In some embodiments, the first processing card is further configured to group the detection data; and the first processing card includes a plurality of first processing sub-cards, each of the plurality of first processing sub-cards is configured to determine the target event based on each grouped detection data.

In some embodiments, the PET system further includes a cable bundle covering cables between the first processing sub-card and detector modules corresponding to the each grouped detection data.

In some embodiments, the second processing card is further configured to group the target event; and the second processing card includes a plurality of second processing sub-cards, each of the plurality of second processing sub-cards is configured to process each grouped target event.

In some embodiments, a switching card is provided between the plurality of first processing sub-cards and the plurality of second processing sub-cards, the switching card is configured to dynamically route target events determined by the plurality of first processing sub-cards.

In some embodiments, the second processing card further includes a scheduling card configured to guide actions of the first processing card and the second processing card based on the detection data and operation information of the first processing card and the second processing card.

In some embodiments, the PET system further includes a preprocessing card communicatively connected to the first processing card and the second processing card, and configured to preprocess the detection data.

In some embodiments, the PET system further includes a data intermediate station communicatively connected to the multiple detector rings and the plurality of first processing cards, and configured to transmit data from the multiple detector rings to the plurality of first processing cards actively or passively.

In some embodiments, the PET system further includes a frame, and the multiple detector rings are sequentially arranged along an axial direction of the frame.

One or more embodiments of the present disclosure provide a PET system. The PET system comprises multiple detector rings, at least one data processing card and at least one coincidence event card. Each of the multiple detector rings includes a plurality of detector modules and have an axial index respectively. The at least one data processing card is communicatively connected to the multiple detector rings, wherein the data processing card is configured to receive detection data with different axial indexes from the multiple detector rings, and determine a target event based on the detection data. The at least one coincidence event card is configured to determine a coincidence event pair by processing the target event, wherein the coincidence event pair at least includes coincidence events detected by the multiple detector rings.

In some embodiments, an axial dimension of the each of the multiple detector rings is greater than or equal to 15 cm, a length of an axial field of view of the PET system is greater than or equal to 60 cm.

In some embodiments, the coincidence event pair is determined by detection data received by detector modules respectively belonging to two adjacent detector rings, or coincidence event pair is determined by detection data received by detector modules respectively belonging to two non-adjacent detector rings.

One or more embodiments of the present disclosure provide a PET system. The PET system comprises multiple detector rings and a processing card. Each of the multiple detector rings includes a plurality of detector modules, wherein the multiple detector rings are arranged side by side along an axial direction of the PET system to form an accommodation cavity. The processing card is communicatively connected to the multiple detector rings, and is configured to receive detection data from the multiple detector rings; determine a target event based on a target single event, detected by detector modules at a same circumferential position across the multiple detector rings; and determine a coincidence event pair by processing the target event.

In some embodiments, the processing card includes a first processing card and a second processing card. The first processing card is communicatively connected to the multiple detector rings, and configured to determine the target event based on the target single event. The second processing card is communicatively connected to the first processing card, configured to determine the coincidence event pair by processing the target event.

In some embodiments, the first processing card is further configured to group the target single event; and the first processing card includes a plurality of first processing sub-cards, each of the plurality of first processing sub-cards is configured to determine the target event based on each grouped target single event.

In some embodiments, the PET system further includes a cable bundle covering cables between the first processing sub-card and detector modules corresponding to the each grouped target single event.

In some embodiments, the PET system further includes a data intermediate station communicatively connected to the multiple detector rings and the first processing card, and configured to transmit data from the multiple detector rings to the first processing card actively or passively.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

### BRIEF DESCRIPTION OF THE DRAWINGS

The present disclosure is further describable in terms of exemplary embodiments. These exemplary embodiments are describable in detail with reference to the drawings. These embodiments are non-limiting exemplary embodiments, in which like reference numerals represent similar structures throughout the several views of the drawings, and wherein:
FIG. 1 is a schematic diagram illustrating an exemplary PET system according to some embodiments of the present disclosure;
FIG. 2 is a schematic diagram illustrating an exemplary structure of a short-axial PET imaging device according to some embodiments of the present disclosure;
FIG. 3 is a schematic diagram illustrating an exemplary structure of a long-axial PET imaging device according to some embodiments of the present disclosure;
FIG. 4 is a schematic diagram illustrating an exemplary structure of a processing device according to some embodiments of the present disclosure;
FIG. 5 is a schematic diagram illustrating an exemplary structure of a processing device according to some embodiments of the present disclosure;
FIG. 6 is a block diagram illustrating an exemplary PET system according to some embodiments of the present disclosure;
FIG. 7 is a schematic diagram illustrating an exemplary structure of detector rings according to some embodiments of the present disclosure;
FIG. 8 is a schematic diagram illustrating an exemplary structure of a PET system according to some embodiments of the present disclosure;
FIG. 9 is a schematic diagram illustrating an exemplary structure of a PET system according to some embodiments of the present disclosure;
FIG. 10 is a schematic diagram illustrating an exemplary structure of a PET system according to some embodiments of the present disclosure;
FIG. 11 is a schematic diagram illustrating an exemplary structure of a PET system according to some embodiments of the present disclosure;
FIG. 12 is a schematic diagram illustrating an exemplary structure of a PET system according to some embodiments of the present disclosure;
FIG. 13 is a flowchart illustrating an exemplary process for performing coincidence determination according to some embodiments of the present disclosure;
FIG. 14 is a schematic diagram illustrating an exemplary structure of a chip according to some embodiments of the present disclosure; and
FIG. 15 is a schematic diagram illustrating an exemplary internal structure of a computer device according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high level, without detail, in order to avoid unnecessarily obscuring aspects of the present disclosure. Various modifications to the disclosed embodiments will be readily apparent to those skilled in the art, and the general principles defined herein may be applied to other embodiments and applications without departing from the spirit and scope of the present disclosure. Thus, the present disclosure is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this disclosure, specify the presence of stated features, integers, steps, operations, elements, and/or components, but do not preclude the presence or addition of one or more other features, integers, steps, operations, elements, components, and/or groups thereof. It may be understood that the terms "system," "unit," "module," and/or "block" used herein are one method to distinguish different components, elements, parts, sections, or assemblies of different levels in ascending order. However, the terms may be displaced by another expression if they achieve the same purpose.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

The flowcharts used in the present disclosure illustrate operations that systems implement according to some embodiments of the present disclosure. It is to be expressly understood, the operations of the flowcharts may be implemented not in order. Conversely, the operations may be implemented in inverted order, or simultaneously. Moreover, one or more other operations may be added to the flowcharts. One or more operations may be removed from the flowcharts.

An object in the present disclosure may be biological or non-biological. For example, the object may include a patient, a man-made object, etc. As another example, the object may include a specific portion, organ, and/or tissue of the patient. For example, the object may include the head, the neck, the thorax, the heart, the stomach, a blood vessel, soft tissue, a tumor, nodules, or the like, or any combination thereof.

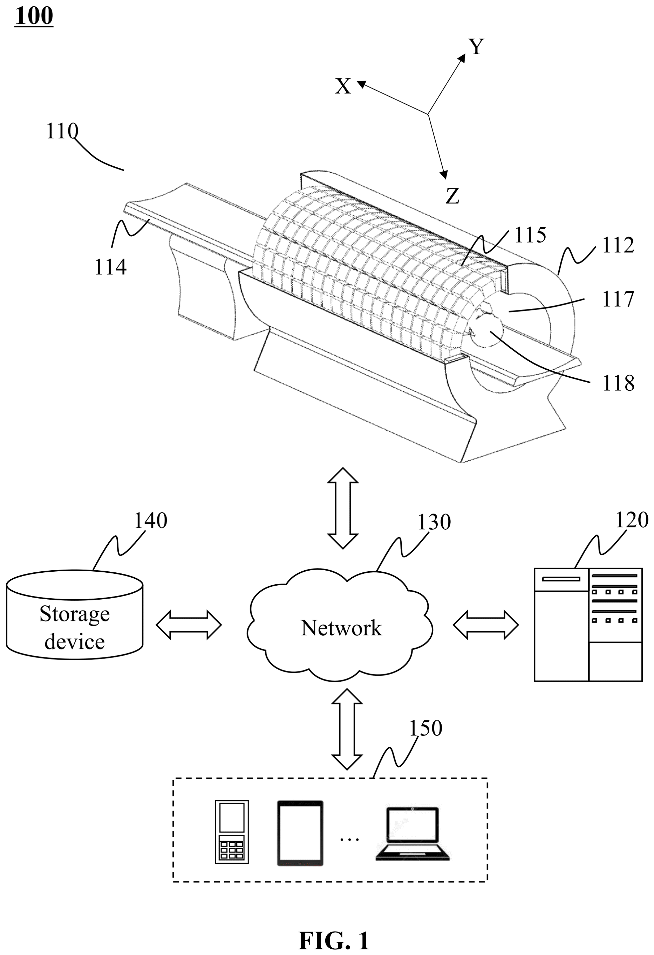

FIG. 1 is a schematic diagram illustrating an exemplary PET system according to some embodiments of the present disclosure. As illustrated in FIG. 1, a PET system 100 may include a PET imaging device 110, a processing device 120, a network 130, a storage device 140, and a terminal device 150. The components of the PET system 100 may be connected in various ways. In some embodiments, the PET imaging device 110, the processing device 120, the storage device 140, and the terminal device 150 are connected to and/or communicate with each other via a wireless connection, a wired connection, or a combination thereof.

The PET imaging device 110 is a medical device configured to detect a distribution of a positron-emitting radionuclide tracer in an object to generate images that reflect molecular metabolism, physiological function, and pathological status of the object. In some embodiments, the PET imaging device 110 may include a single modality device and/or a multi-modality device. The single modality device may include, for example, a PET device, a single-photon emission computed tomography (SPECT) device, etc. The multi-modality device may include, for example, a positron emission tomography-magnetic resonance imaging (PET-MRI) device, a single-photon emission computed tomography-magnetic resonance imaging (SPECT-MRI) device, a positron emission tomography-computed tomography (PET-CT) device, etc. For illustration purposes, the present disclosure describes a PET device.

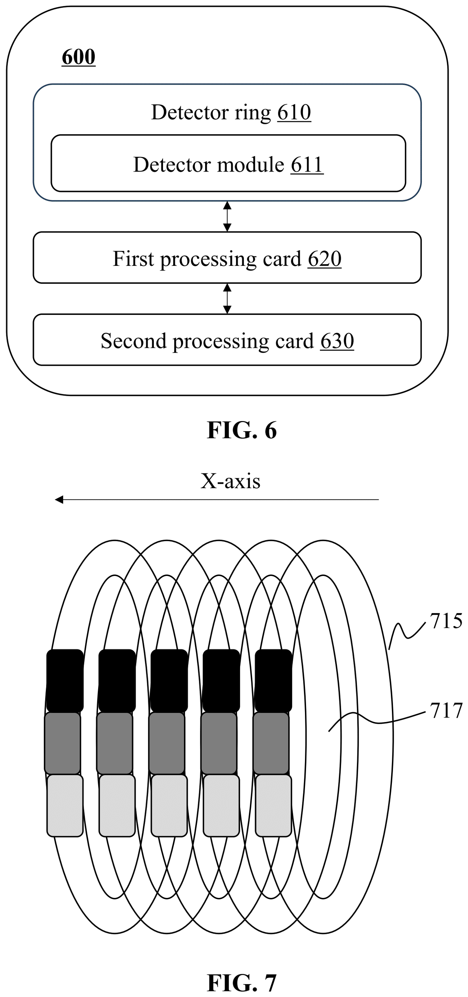

In some embodiments, the PET imaging device 110 may include a frame 112, a couch 114, and multiple detector rings 115 arranged along an axial dimension (e.g., an X-axis direction illustrated in FIG. 1) of the PET imaging device 110, which form a field of view (FOV) of the PET imaging device 110. The frame 112 may support the multiple detector rings 115. The couch 114 may be used to support an object 118 to be scanned. Each of the multiple detector rings 115 may include a plurality of detector modules arranged along the circumference of the detector ring. More descriptions of the detector rings 115 and the detector modules may be found elsewhere in the present disclosure (e.g., FIGs. 6-7 and the descriptions thereof).

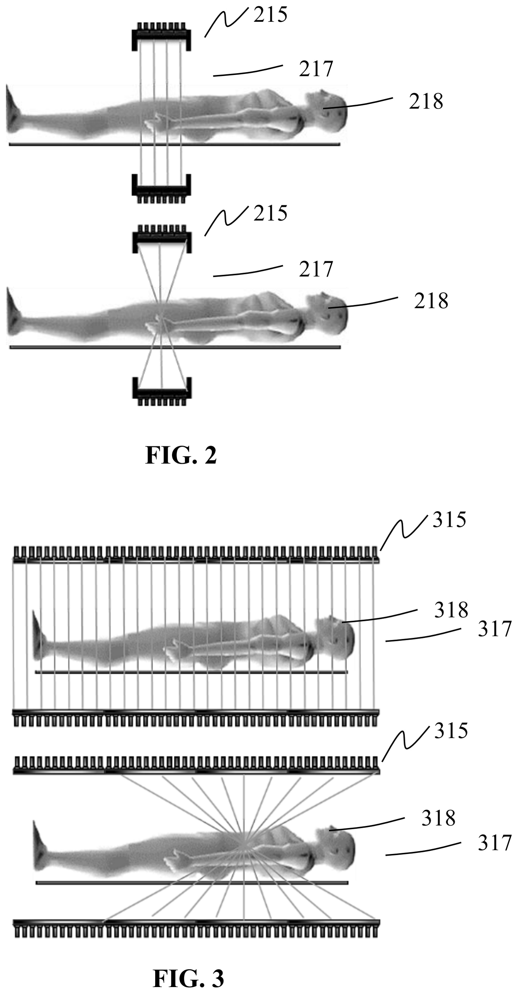

Based on a length of an axial field of view, the PET imaging device can be categorized into a short-axial PET imaging device and a long-axial PET imaging device. More descriptions of the short-axial PET imaging device and the long-axial PET imaging device may be found elsewhere in the present disclosure (e.g., FIGs. 2-3 and the descriptions thereof).

In some embodiments, the object 118 may be moved by moving the couch 114 within a detection region 117 of the PET imaging device 110. In some embodiments, the couch 114 may move along the axial dimension of the PET imaging device 110. For example, the couch 114 may move along the positive X-axis direction or the negative X-axis direction. As another example, the couch 114 may move back and forth along the X-axis direction.

In some embodiments, before a PET scan, the object 118 may be injected with a tracer species. The tracer species may refer to a radioactive substance that decays and emits positrons. In some embodiments, the tracer species may be radioactively marked radiopharmaceutical, which is a drug having radioactivity and is administered to the object 118. For example, the tracer species may include fluorine-18 (18F) fluorodeoxyglucose (FDG), etc. During the scanning, pairs of photons (e.g., gamma photons) may result from the annihilation of positrons originating from the tracer species in the object 118. A pair of photons may travel in opposite directions. At least a part of the pairs of photons may be detected and/or registered by the detector module. A coincidence event may be recorded when a pair of photons generated by the positron-electron annihilation is detected within a coincidence time window (e.g., within 6 ns to 12 ns).

The processing device 120 may process data and/or information obtained from other device(s) or system component(s) (e.g., the PET imaging device 110, the storage device 140, the terminal device 150) to perform one or more functions described in some embodiments of the present disclosure based on the data, information and/or a processing result. In some embodiments, the processing device 120 is a computer, a user console, a single server, or a server group. The server group may be centralized or distributed. In some embodiments, the processing device 120 may include a central processing unit (CPU), a digital signal processor (DSP), a system on a chip (SoC), a microcontroller unit (MCU), or the like, or any combination thereof. In some embodiments, the processing device 120 may be local or remote. In some embodiments, the processing device 120 may be implemented on a cloud platform. In some embodiments, the processing device 120 or a portion of the processing device 120 may be integrated into the PET imaging device 110 and/or the terminal device 150. It should be noted that the processing device 120 in the present disclosure may include one or multiple processors. Thus operations and/or method steps that are performed by one processor may also be jointly or separately performed by the multiple processors.

The network 130 may include any suitable network that can facilitate the exchange of information and/or data for the PET system 100. In some embodiments, one or more components of the PET system 100 (e.g., the PET imaging device 110, the processing device 120, the storage device 140, the terminal device 150, etc.) may communicate information and/or data with one or more other components of the PET system 100 via the network 130. In some embodiments, the network 130 may be one or more of a wired network and a wireless network.

The storage device 140 may store data, instructions, and/or any other information. In some embodiments, the storage device 140 may store data obtained from the PET imaging device 110, the processing device 120, and/or the terminal device 150. In some embodiments, the storage device 140 may store data and/or instructions that the processing device 120 may execute or use to perform exemplary methods described in the present disclosure. In some embodiments, the storage device 140 may include a mass storage device, a removable storage device, a volatile read-and-write memory, a read-only memory (ROM), or the like, or a combination thereof. In some embodiments, the storage device 140 may be implemented on a cloud platform. In some embodiments, the storage device 140 may be part of the PET imaging device 110, the processing device 120, or the terminal device 150.

The terminal device 150 may be configured to enable user interactions between a user and the PET system 100. In some embodiments, the terminal device 150 may be connected to and/or communicate with the PET imaging device 110, the processing device 120, and/or the storage device 140. In some embodiments, the terminal device 150 may include a mobile device, a tablet computer, a laptop computer, or the like, or a combination thereof. In some embodiments, the terminal device 150 may be part of the processing device 120 and/or the PET imaging device 110.

It should be noted that the above description is intended to be illustrative, and not to limit the scope of the present disclosure. Many alternatives, modifications, and variations will be apparent to those skilled in the art. The features, structures, methods, and characteristics of the exemplary embodiments described herein may be combined in various ways to obtain additional and/or alternative exemplary embodiments. In some embodiments, the PET system 100 may include one or more additional components and/or one or more components described above may be omitted. Additionally or alternatively, two or more components of the PET system 100 may be integrated into a single component. For example, the processing device 120 may be integrated into the PET imaging device 110. As another example, a component of the PET system 100 may be replaced by another component that can implement the functions of the component. However, those variations and modifications do not depart from the scope of the present disclosure.

As described in connection with FIG. 1, the PET system is categorized into a short-axial PET imaging device and a long-axial PET imaging device. FIGs. 2 and 3 are schematic diagrams illustrating exemplary structures of a short-axial PET imaging device and a long-axial PET imaging device, respectively, according to some embodiments of the present disclosure. As illustrated in FIGs. 2 and 3, when a PET device is used for medical imaging, an object (e.g., an object 218 illustrated in FIG. 2, an object 318 illustrated in FIG. 3) is located in a detection region (e.g., a detection region 217 illustrated in FIG. 2, a detection region 317 illustrated in FIG. 3) formed by the multiple detector rings (e.g., multiple detector rings 215 illustrated in FIG. 2, multiple detector rings 315 illustrated in FIG. 3) of the PET device. Detection data detected by the PET device includes not only signals (also referred to as intra-ring events, as illustrated by parallel response lines in FIGs. 2 and 3) between detector modules located within a same detector ring but also a large count of cross signals (also referred to as inter-ring events, as illustrated by cross response lines in FIGs. 2 and 3) between detector modules located within different rings.

A structural difference between the short-axial PET imaging device and the long-axial PET imaging device lies in an axial length (i.e., a length in a direction from the head to feet of the object) of the detector rings. The short-axial PET imaging device has a short-axial length and a lower count (e.g., 20-40) of detector rings. The short-axial PET imaging device usually requires multi-bed scanning combined with image stitching technology to achieve local or whole-body imaging. With a relatively compact overall structure and a low cost, the short-axial PET imaging device is suitable for a scenario such as routine clinical examination and medical institution with cost constraints. The long-axial PET imaging device can achieve whole-body or large-scale synchronous dynamic imaging by significantly increasing a count (typically 100-300) of axially stacked detector rings. The long-axial PET imaging device has high sensitivity and is suitable for a scenario such as whole-body tumor screening and pharmacokinetic research. However, an expansion of axial field of view poses severe technical challenges. A radioactive signal (also referred to as single event data) emitted by the object and detected by the long-axial PET imaging device per unit time increases geometrically, and a count of coincidence event pairs generated accordingly also increases dramatically, placing enormous pressure on a real-time data processing of a back-end electronic system (e.g., the processing device 120).

For example, as illustrated in FIG. 2, assume that the long-axial PET imaging device includes 8 detector rings, and the detector rings are indexed as 0, 1, 2, ..., 7. Coincidence event pairs to be processed by the processing device 120 for each detector ring include not only intra-ring coincidence event pairs (e.g., 0-0, 1-1, ..., 7-7), but also all possible inter-ring coincidence event pairs (e.g., 0-1, 0-2, ..., 1-0, ..., 7-6, etc.). This means that a processing capability of the processing device 120 required for the 8-ring long-axial PET imaging device is far greater than that for a single-ring PET imaging device.

To meet demands of massive data processing, a distributed processing approach is commonly adopted to process detection data detected by the long-axial PET imaging device. For example, a plurality of processing cards (also referred to as counting boards (CCBs) or coincidence processing cards) are allocated to different detector rings to collaboratively process the detection data.

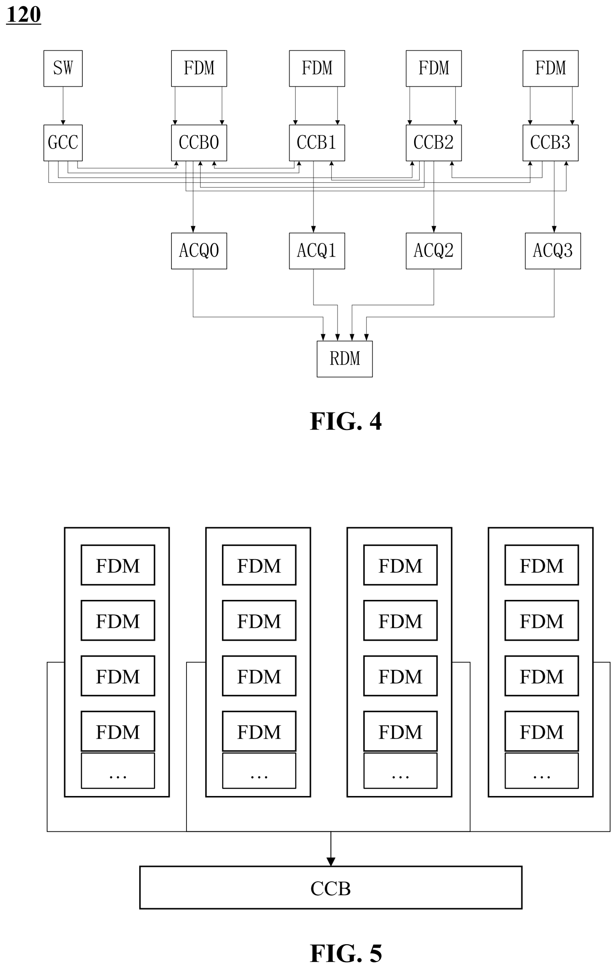

FIG. 4 is a schematic diagram illustrating an exemplary structure of a processing device according to some embodiments of the present disclosure. As illustrated in FIG. 4, the processing device 120 corresponding to the long-axial PET imaging device may include front-end detector modules (FDMs), a supervisor workstation (SW), a control unit (GNU Compiler Collection, GCC), CCBs, data acquisition cards (ACQs), and a raw data memory (RDM).

The FDMs are front-end electronic units directly connected to detector modules of the long-axial PET imaging device, and configured to convert physical detection events into transmittable and processable detection event data. For example, the FDMs receive weak analog signals generated by crystals of the detector modules, perform amplification, shaping, and extraction of time and energy information, and finally digitize the analog signals into standardized single event data containing information such as a timestamp, a detector module position, and an energy value.

The SW is configured to coordinate an entire scanning process of the long-axial PET imaging device. For example, the SW sends a macro control instruction (e.g., an instruction to start scanning, an instruction to set parameters, etc.) to the long-axial PET imaging device, and receives, processes, and displays a final result.

The GCC is located between the SW and a subordinate unit (e.g., the CCB, the FDM, etc.), and configured to provide real-time and precise collaborative control of hardware. For example, the GCC receives the macro control instruction from the SW, parses and translates it into a specific timing control signal, a configuration parameter, etc., and distributes these signals, parameters, etc., to the subordinate unit.

The CCBs are configured to process the detection data. For example, the CCBs are configured to receive a single event data stream from the FDMs and execute a preset algorithm, etc. In a distributed architecture, each detector ring is equipped with a respective CCB. Detection data generated by a plurality of detector modules on the detector ring converges to a corresponding CCB (via the FDM). The CCB first determines coincidence event pairs (also referred to as an intra-ring coincidence determination or an intra-ring coincidence processing) based on detection data from the detector ring to which the CCB belongs. Because there is a transmission of detection data between the CCBs (as schematically illustrated by data transmission from CCB3 to CCB2 in FIG. 4), the CCB further determines coincidence event pairs (also referred to as an inter-ring coincidence determination or an inter-ring coincidence processing) based on detection data transmitted to the CCB from other detector rings. More descriptions of the CCB may be found elsewhere in the present disclosure.

The ACQs are located at a backend of the CCBs, and configured to address rate and interface matching issues between data output from the CCB and a computer storage system, thereby enabling reliable and continuous data acquisition. The RDM is located at a backend of the ACQs, and configured to store raw or preliminarily processed data. The aforementioned components (the FDMs, the SW, the GCC, the CCBs, the ACQs, and the RDM) implement a complete functional chain from "data detection" to "event recording". Specifically, the FDMs are responsible for signal digitization; the GCC provides global coordination under instructions from the SW; the CCBs perform core coincidence processing; the ACQs are responsible for data aggregation and transmission; and the final data is stored in the RDM.

A count of the CCBs depends on a count of detector rings in the long-axial PET imaging device or the processing capability of the CCBs. As illustrated in FIG. 4, a plurality of CCBs (e.g., CCB0-CCB3) are used to perform coincidence determination (e.g., intra-ring coincidence determination and inter-ring coincidence determination) on detection data detected by four detector rings in the long-axial PET imaging device. Each CCB can process detection data of a detector ring corresponding to the CCB, and perform inter-ring coincidence determination on detection data transmitted from other detector rings.

In an existing PET data processing flow, to reduce resource consumption of backend coincidence determination, a "circumferential merging" strategy may also be adopted as a data preprocessing approach to compress an amount of single event data from a plurality of adjacent detector modules within a same detector ring.

FIG. 5 is a schematic diagram illustrating an exemplary structure of a processing device according to some embodiments of the present disclosure. As illustrated in FIG. 5, each column represents a detector ring. Each detector ring includes a plurality of detector modules. When a CCB receives detection data, the CCB may perform circumferential merging on the detection data. The circumferential merging refers to merging detection data of a plurality of detector modules (usually adjacent modules) on a same detector ring. Specifically, the circumferential merging follows the following steps. First, a time granularity (e.g., 2 ns, etc.) is set as a minimum time window for discretizing an event stream. Each single event is assigned to a time granularity according to its occurrence time. If only one single event occurs within a time granularity, the single event is directly retained; if a plurality of single events occurs within the time granularity, only one of them is retained according to a preset rule (which may be set based on experience or requirements, e.g., random selection, etc.), and the remaining events are discarded, thereby achieving data volume compression.

Merely by way of example, assume that a time granularity is 1 second, detection data of a detector module A and a detector module B are merged. Single events from the detector module A occur at t=1 s and t=3 s, and a single event from the detector module B occurs at t=2 s. Within the time granularity at t=1 s, only the detector module A has a single event, so the single event from the detector module A at t=1 s is retained. Similarly, within the time granularity at t=2 s, only the detector module B has a single event, so the single event from the detector module B at t=2 s is retained; within the time granularity at t=3 s, only the detector module A has a single event, so the single event from the detector module A at t=3 s is retained. If each of the detector module A and the detector module B generates a single event within the same time granularity at t=2 s, a merging rule is triggered. The CCB may randomly select the single event from the detector module A or the single event from the detector module B at t=2 second to retain, and discard the other single event. Therefore, a merged output event stream includes only one single event at t=2 second.

As illustrated in FIG. 5, the long-axial PET imaging device may include 4 detector rings, each detector ring includes a plurality of detector modules. If each detector ring includes 6 detector modules, there are 24 detector modules in total. To perform merging, the CCB may divide all 24 detector modules into a plurality of groups according to their circumferential position indexes. For example, detector modules of each detector ring are numbered 1-6. Detector modules with index numbers 1 and 2 in the 4 detector rings are classified into a first group, detector modules with index numbers 3 and 4 are classified into a second group, and detector modules with index numbers 5 and 6 are classified into a third group. For each group, the CCB performs the merging operation described above on a corresponding data stream of the group. That is, within each time granularity, events occurring in all detector modules of the group are screened, so that each group outputs at most one single event within each time granularity. In this way, a total count of events subsequently required for coincidence determination is effectively reduced, thereby reducing requirements of the PET system for processing resources and data transmission bandwidth.

It is understandable that, although distributed processing and a circumferential merging strategy are introduced, certain limitations still exist. First, although tasks are distributed to a plurality of CCBs through distribution, each CCB is still required to perform complete coincidence processing, resulting in significant overall resource overhead. Second, the plurality of CCBs are required to cooperate with each other, a count of components and cables is large, a system architecture is complex, and reliability and stability are low. Third, to complete the inter-ring coincidence determination, a large amount of single event data needs to be transmitted between the CCBs. The transmission has extremely high requirements on link bandwidth, synchronization, and stability. Once data delay, loss, or mismatch occurs, it may lead to persistent abnormalities in the inter-ring coincidence determination, thereby affecting final imaging quality.

To overcome the above defects, the embodiments of the present disclosure provide a PET system. FIG. 6 is a block diagram illustrating an exemplary PET system according to some embodiments of the present disclosure. In some embodiments, as illustrated in FIG. 6, a PET system 600 may include a detector ring 610, a first processing card 620, and a second processing card 630. In some embodiments, functions corresponding to the first processing card 620 and the second processing card 630 may be performed by the processing device 120.

The detector ring 610 is a physical structure of the PET system for data acquisition. A design and arrangement of the detector ring 610 directly determine an axial field of view and an imaging performance of the PET system.

In some embodiments, the PET system 600 may include multiple detector rings 610 arranged side by side along an axial direction of the PET system. FIG. 7 is a schematic diagram illustrating an exemplary structure of detector rings according to some embodiments of the present disclosure. As illustrated in FIG. 7, each detector ring 715 may be a ring structure. Multiple detector rings are closely stacked along the axial direction (i.e., an X-axis direction in FIG. 7) of the multiple detector rings to form a detection region 717 (also referred to as an accommodation cavity for accommodating an object) of the PET imaging device. An axial length of the PET imaging device depends on a count of the stacked detector rings in the X-axis direction.

In some embodiments, each detector ring 610 includes the plurality of detector modules 611 arranged along a circumferential direction of the detector ring 610. As illustrated in FIG. 7, each detector ring is a complete 360-degree ring structure, which is formed by splicing a plurality of detector modules along the circumferential direction (corresponding to gray rectangles in FIG. 7) of the detector ring. It should be noted that the 3 detector modules in FIG. 7 are only for illustration. In practical applications, the plurality of detector modules are closely arranged to cover an entire 360-degree circumference of the detector ring. Each detector module usually covers a circumferential angle of from several degrees to over ten degrees. In some embodiments, in the same PET imaging device, the count of detector modules 611 on each detector ring 610 is the same, and their circumferential positions are aligned.

In some embodiments, the detector module 611 includes a scintillation crystal and a photoelectric conversion device. The scintillation crystal is a sensing unit configured to convert incident 511 keV gamma photons into visible light. The photoelectric conversion device is configured to convert a visible light signal into an electrical signal for subsequent processing.

In some embodiments, the PET system 600 may further include a frame (e.g., the frame 112). The multiple detector rings 610 are arranged in sequence along an axial direction of the frame. The frame is configured to support the multiple detector rings 610. It is understandable that, from a physical implementation perspective, the multiple detector rings 610 are installed on the frame. Designs of different PET systems are different. For example, a frame may carry a single detector ring or multiple detector rings. To focus on a data processing logic of some embodiments of the present disclosure and avoid introducing unnecessary structural details, subsequent descriptions will uniformly use the detector ring as a core structural unit, without deliberately distinguishing an assembly relationship between the detector ring and the frame.

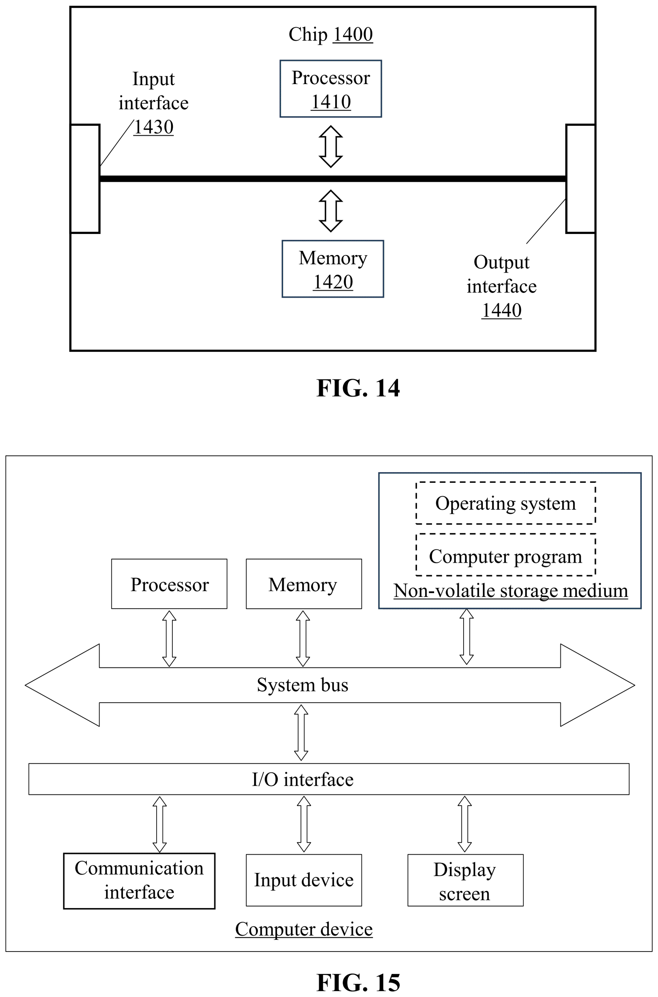

The first processing card 620 refers to a component for processing the detection data. The first processing card 620 may be implemented in a form of a printed circuit board (PCB) (integrated with an electronic component such as a processor, a memory, and an interface), a chip (e.g., a chip 1400 illustrated in FIG. 14), or the like. In some embodiments, the PET system 600 may include the first processing card 620. The first processing card 620 is communicatively connected to the multiple detector rings 610, and configured to receive detection data with a same circumferential index, and determine a target event based on the detection data. The circumferential index is based on a circumferential position. In some embodiments, the PET system 600 may include a plurality of first processing cards 620.

The detection data refers to a data set directly output by the detector ring and is composed of a plurality of single events. The single event refers to complete data recorded when a single detector module detects one gamma photon during a PET imaging process. In some embodiments, the single event may include an event occurrence time (also referred to as a timestamp), an occurrence position (i.e., position information of a detector module and/or a detector ring, also referred to as a module position identifier), and energy information (also referred to as an energy value).

The target event refers to a representative event retained after merging and/or selecting all single events from detector modules at the same circumferential position on multiple detector rings within a set time granularity.

In some embodiments, the first processing card 620 may process the detection data based on a preset processing rule, a machine learning model, a preset algorithm, or the like, to determine the target event.

In some embodiments, the first processing card 620 may determine the target event based on a target single event within a same time granularity in the detection data. The target single event refers to at least one single event detected by the detector modules at the same circumferential position on the multiple detector rings.

In some embodiments, for a target single event within the same time granularity in the detection data, in response to the target single event including one single event, the first processing card 620 may designate the single event as the target event; in response to the target single event including at least two single events, the first processing card 620 may select one from the at least two single events as the target event. Unselected single event(s) is(are) discarded and is not involved in any subsequent operations or calculations.

In some embodiments, before determining the target event, the first processing card 620 may select, randomly or based on a preset rule, the one from the at least two single events as the target event. Random selection refers to selecting one from the at least two single events in a random manner as the target event. Selection based on a preset rule refers to selecting one from the at least two single events according to a predefined rule (e.g., alternating selection among different detector rings) to avoid long-term bias towards a specific detector ring or detector module. Determining the target event randomly or based on the preset rule can ensure statistical uniformity in event discarding and reduce a risk of systematic bias.

In some embodiments, for each of the at least two single events, the first processing card 620 may determine a weight of the single event based on at least one of a reliability of time information of the single event, energy information of the single event, and related weighting parameters of a detector ring corresponding to the single event. The first processing card 620 may select, based on weights of the at least two single events, the one from the at least two single events as the target event.

The weight of the single event refers to a numerical value or rank assigned to each single event, used to characterize a relative priority of the single event as a representative event in the target event. The reliability of time information of the single event reflects the measurement accuracy of an occurrence time of the single event, and may be characterized by at least one indicator from a reliability of time interpolation, a trigger edge quality, and a time quantization residual. The aforementioned data (i.e., the reliability of time interpolation, the trigger edge quality, and the time quantization residual) are data fields contained in raw detection data or quality indication information generated during a generation of the raw detection data, and do not require additional determination or calculation. The energy information of the single event refers to an energy value corresponding to the single event, and is configured to reflect whether the single event is close to a target energy or satisfies an energy selection requirement. The related weighting parameters of the detector ring corresponding to the single event are configured to reflect differences among different detector rings in statistical characteristics, stability, or operational status. The related weighting parameters of the detector ring corresponding to the single event may be determined based on a fluctuation in a single event count rate and a load of the detector ring within a historical time period. For example, a small fluctuation in the single event count rate indicates stable operation; a low load of the detector ring indicates a better load status, the corresponding weight parameter related to the detector ring is high.

In some embodiments, the first processing card 620 may perform normalization and then weighted processing on the aforementioned information (i.e., the reliability of time interpolation, the trigger edge quality, and the time quantization residual), with weights preset based on experience or requirements, to determine the weight of the single event. The first processing card 620 may also determine the weight of the single event using a preset table or model.

In some embodiments, among the at least two single events, the first processing card 620 may select the single event with a higher weight as the target event based on their respective weights, to prioritize retention of higher-quality event and improve the reliability of the target event.

In some embodiments, before determining the target event, the first processing card 620 may determine a single event count within a target time window based on the detection data detected by the detector modules at the same circumferential position, wherein the time granularity used by the first processing card 620 to determine the target event based on the target single event is within the target time window. In response to the single event count being less than a preset count threshold, the first processing card 620 does not determine the target event based on the target single event within the same time granularity in the detection data, but sends the detection data to the second processing card 630 for processing the detection data to determine the coincidence event pair. In response to the single event count being greater than the preset count threshold, the first processing card 620 determines the target event based on the target single event within the same time granularity in the detection data.

The target time window refers to a time range used to evaluate an event density. A length of the target time window may be greater than the time granularity used by the first processing card 620 to determine the target event based on the target single event, to reflect an event density level over a time period.

The single event count refers to a total count of single events detected by detector modules at the same circumferential position across all detector rings within the target time window. The single event count is configured to characterize an event generation density of the PET imaging device within the target time window.

The preset count threshold refers to a count threshold used to define a "low event density state" (indicating that real-time data processing pressure of the PET system is relatively low) and a "high event density state" (indicating that real-time data processing pressure of the PET system is relatively high). The threshold may be preset based on the processing capability of the PET system or experience.

The coincidence event pair refers to a pair of events determined to potentially originate from a same annihilation event. It is understandable that the PET imaging is based on detecting paired gamma photons. However, from a perspective of the back-end electronics system, it processes only two digitized event signals that are close in time, meet energy requirements, and are spatially positioned opposite. The back-end electronics system cannot prove that the two photons necessarily originate from a same annihilation point within a body, only infers, based on preset physical criteria (e.g., a time window, an energy window, etc.), that the two events are highly likely to be associated with the same annihilation.

It is understandable that, in response to the single event count being less than the preset count threshold, the first processing card 620 may determine that a current state of the PET system is the low event density state. The first processing card 620 does not determine the target event based on the target single event within the same time granularity in the detection data (i.e., bypasses the processing of the detection data by the first processing card 620) and directly sends the detection data corresponding to the circumferential position to the second processing card 630 for processing. In response to the single event count being greater than the preset count threshold, the first processing card 620 may determine that the current state of the PET system is the high event density state. The first processing card 620 determines the target event based on the target single event within the same time granularity in the detection data (i.e., normally determines the target event through the first processing card 620). Through event density state determination, unnecessary processing can be avoided when the PET system is in the low event density state, thereby reducing information loss and lowering processing overhead.

In some embodiments, the aforementioned operation of comparing the single event count with the preset count threshold is performed at every preset time length.

The preset time length refers to a time interval parameter used to periodically trigger the aforementioned operation of comparing the single event count with the preset count threshold. The parameter may be set according to stability of the PET system or dynamic change of the PET scanning process. During an entire scanning process of the PET system, the first processing card 620 may periodically re-evaluate whether a current single event count exceeds the preset count threshold. Based on the determination result, the first processing card 620 dynamically selects whether to initiate determination of the target event for the circumferential position. It is understandable that the process of determining the target event is not performed continuously throughout the entire scanning process. Instead, the process is dynamically enabled or disabled according to real-time changes in event density, thereby achieving adaptive matching of a data processing strategy with the scanning process and improving overall processing flexibility.

In some embodiments, the time granularity may be determined based on at least one of a count of single events detected, per unit time, by the detector modules at the same circumferential position across the multiple detector rings or a multiple data occurrence rate within a granularity.

The count of single events detected, per unit time, by the detector modules at the same circumferential position across the multiple detector rings characterizes an event input intensity of the detector modules, reflecting an overall density level of the detection data, and it itself does not depend on a value of the time granularity. The multiple data occurrence rate within a granularity refers to a ratio of a count of time granularities in which at least two single events occur to a total count of time granularities. For example, using 2 ns as a time granularity, within a continuous statistical duration of 10 ns, the first processing card 620 performs 5 target event determination operations (10 ns/2 ns=5). Assuming that in 4 of these 5 operations, two or more single events occur, the multiple data occurrence rate within the granularity (2 ns) is 4/5, i.e., 80%. This means that in up to 80% of time periods, the first processing card 620 has to discard some single events and retain only one of them based on the target event determination operation. An excessively high multiple data occurrence rate within the granularity leads to a significant loss of potentially effective information, which may negatively impact statistical accuracy and quantitative accuracy of subsequent image reconstruction. It is understandable that the multiple data occurrence rate within the granularity is related to the time granularity. The larger the time granularity is, the higher the multiple data occurrence rate within the granularity is.

In some embodiments, the first processing card 620 may first determine an initial time granularity. The initial time granularity refers to a plurality of selectable time granularity values preset or dynamically generated, and is configured to evaluate target event determination effects under different time granularities. The first processing card 620 may determine the initial time granularity based on a count of single events detected, per unit time, by the detector modules at the same circumferential position across the multiple detector rings, such that an expected count of single events within the initial time granularity falls within a preset range. The two may be negatively correlated. That is, the greater the count of single events, the smaller the initial time granularity, so that an average count of events expected to occur within a single time granularity is maintained within a low target range (e.g., close to 1 event), thereby reducing a risk of excessive event density that may lead to high conflict and high information discarding.

Further, the first processing card 620 may determine the multiple data occurrence rate within the granularity based on the initial time granularity, and dynamically optimize or adjust the initial time granularity to determine the time granularity. The first processing card 620 may run the target event determination process under the initial time granularity and calculate a current multiple data occurrence rate within the initial time granularity. If the current multiple data occurrence rate within an initial time granularity is higher than a preset threshold (which may be set based on experience or requirements), it indicates that the initial time granularity is too large, causing too many events to occur within the same time window and be discarded. The first processing card 620 may decrease the initial time granularity. If the current multiple data occurrence rate within the initial time granularity is lower than the preset threshold, it indicates that the initial time granularity is too small. The first processing card 620 may increase the initial time granularity. By repeating the process, the first processing card 620 dynamically adjusts the initial time granularity until the current multiple data occurrence rate within the initial time granularity is approximately equal to the preset threshold (e.g., a difference between the current multiple data occurrence rate within the initial time granularity and the preset threshold is less than a threshold). The first processing card 620 determines a final time granularity (e.g., designates the initial time granularity as the final time granularity). While ensuring normal progress of the target event determination process, the first processing card 620 controls the multiple data occurrence rate so as to avoid unnecessary information discard.

In some embodiments, the time granularity may be determined based on at least one of a card load or a link bandwidth of the PET system.

The card load refers to a computing resource occupancy of a card that performs target event determination or coincidence event pair determination in a current operating state, for example, a processing utilization rate, a cache occupancy level, or the like.

The link bandwidth refers to a data transmission capability of a data transmission channel between the PET imaging device and back-end processing units, or between different back-end processing units, and is configured to constrain an amount of data that can be transmitted per unit time.

In some embodiments, when the card load is high or the link bandwidth is limited, the processing or transmission capability of the PET system is near a limit. The first processing card 620 may increase the time granularity to reduce an amount of event data that needs to be processed or transmitted per unit time, thereby alleviating resource pressure and ensuring stable operation of the PET system. When the card load is low or the link bandwidth is sufficient, the PET system has a capability to process more data. The first processing card 620 may decrease the time granularity to retain more event information on a finer time scale, which is beneficial for improving count statistical accuracy and time resolution of subsequent imaging. By determining the time granularity based on at least one of the card load or the link bandwidth of the PET system, the time granularity can be matched with the processing and transmission capabilities of the PET system, thereby improving the operational stability of the PET system.

In some embodiments, in response to at least two detector modules at the same circumferential position across the multiple detector rings detecting a plurality of single events within the time granularity, the first processing card 620 may perform fusion on the plurality of single events to obtain a fusion event as a target event.

It is understandable that the fusion refers to a process in which, when a plurality of single events are detected by the at least two detector modules at the same circumferential position across the multiple detector rings within the same time granularity, instead of simply selecting one single event from the plurality of single events and discarding the rest, a new, representative fusion event is generated based on the original information of the plurality of single events (e.g., time, energy, etc.) through a preset fusion algorithm. The fusion event serves as the target event for the circumferential position within the time granularity and participates in subsequent coincidence event pair determination. While generating the representative event, the fusion does not discard the original single event data on which the fusion event is based, allowing the original single event data to still be used for subsequent physical correction, quality assessment, or retrospective analysis, thereby reducing the discard of original information and providing a data basis for subsequent correction and analysis.

In some embodiments, the fusion is a weighted merging, and weights are determined based on at least one of energy or consistency of the plurality of single events detected by the at least two detector modules at the same circumferential position across the multiple detector rings within the same time granularity.

The weighted merging refers to performing a combined calculation on a part of original information of the plurality of single events that constitute the fusion event, according to preset weights, when generating the fusion event. Similar to a single event, the fusion event may also include an event occurrence time (also referred to as a timestamp), an occurrence position (i.e., position information of a detector module and/or a detector ring, also referred to as a module position identifier), and energy information (also referred to as an energy value). In some embodiments, the part of the original information may include the event occurrence time and the energy information, and the occurrence position does not participate in the weighted calculation.

The weights may be determined based on the event occurrence time and the energy information. For example, the first processing card 620 may determine the weights based on the energy of the single events constituting the fusion event, or based on the closeness of the energy values to a target energy value (e.g., 511 keV). The single event with an energy value closer to the target energy value is considered to have a higher probability of originating from a real annihilation reaction (i.e., more likely to be a true coincidence event and contribute more to the fusion result), and therefore may be assigned a higher weight. As another example, the first processing card 620 may determine the weights based on the closeness of the occurrence times of the single events constituting the fusion event to a center moment of the time granularity corresponding to the fusion processing, or based on a temporal consistency of the single event with respect to other single events to be fused. A single event with a higher closeness or a higher consistency may be assigned a higher weight.

To determine the occurrence position of the fusion event, the first processing card 620 may select, according to a preset rule, a representative event from the plurality of original single events as a dominant event. The preset rule may be determined based on factors such as energy magnitude, timestamp quality, or event validity. For example, the preset rule may be to select the single event with an energy value closest to 511 keV as the dominant event. The first processing card 620 may use the detector module position corresponding to the dominant event as the occurrence position of the fusion event.

The second processing card 630 refers to a component for processing the target event. The second processing card 630 may be implemented in a form of a printed circuit board (PCB) (integrated with an electronic component such as a processor, a memory, and an interface), a chip (e.g., the chip 1400 illustrated in FIG. 14), or the like. The second processing card 630 is communicatively connected to the plurality of first processing cards 620 and configured to determine the coincidence event pair by processing the target event.

In some embodiments, the second processing card 630 may determine a candidate coincidence event based on the target event; determine a target event that meets a preset condition from target events, the target events being within a preset time window and corresponding to target detector modules, wherein the target detector modules are positioned opposite or approximately opposite to the detector module corresponding to the candidate coincidence event; designate the target event that meets the preset condition and the candidate coincidence event as a candidate coincidence event pair; and determine the coincidence event pair based on the candidate coincidence event pair.

The candidate coincidence event refers to a target event that serves as a reference object for subsequent coincidence processing. The coincidence processing refers to processing detection data generated by the detector rings to identify detection events corresponding to a pair of γ photons generated by a same positron annihilation. In some embodiments, the second processing card 630 may use any target event as the candidate coincidence event.

The preset time window refers to a time range configured to determine whether two events may originate from a same annihilation, e.g., 10 ns, etc. The target detector module refers to the detector module that is opposite or approximately opposite in spatial position to the detector module corresponding to the candidate coincidence event (preferably using an opposite detector module as the target detector module). The opposite or approximately opposite means that a line connecting the two detector modules passes through the object, thereby meeting a geometric condition for PET imaging. For example, a detector ring has 24 detector modules numbered 1 to 24 in sequence, then detector module 1 is opposite to detector module 13, and is approximately opposite to detector modules 12 and 14.

The preset condition refers to a set of criteria used to determine whether two target events originate from the same positron annihilation event, and the preset condition includes at least one of a time condition, an energy condition, or a position condition. The time condition may be that a difference between the occurrence times of the two target events (e.g., the candidate coincidence event and the target event) is less than a preset time difference threshold, and the preset time difference threshold may be set based on the time resolution capability of the PET system and a coincidence selection requirement. The energy condition may be that the event energies corresponding to the two target events (e.g., the candidate coincidence event and the target event) meet a preset energy condition, and the preset energy condition is configured to screen out scattering events, preferably events having an event energy within a target energy (511 keV) or a target energy window (preset based on experience or requirements). The position condition may be that the detector modules corresponding to the two target events (e.g., the candidate coincidence event and the target event) are opposite or approximately opposite in space, so that a line connecting the two detector modules passes through the object.

In some embodiments, the second processing card 630 may determine the coincidence event pair by performing physical correction on the candidate coincidence event pair. It is understandable that the candidate coincidence event pair may include three types: a true coincidence that originates from the same annihilation event, a scattered coincidence where photons are misjudged as coincident after scattering, and a random coincidence formed by two unrelated photons being accidentally close in time (the latter two may be collectively referred to as noise or false coincidences). The physical correction may include processing methods such as random coincidence correction, scatter correction, etc., which are not limited in the embodiments of the present disclosure. Through the physical correction, more accurate coincidence event pairs can be identified and extracted from the candidate coincidence event pairs.

In some embodiments, in response to existence of the fusion event in the candidate coincidence event or the target event corresponding to the target detector module, the second processing card 630 may calculate a matching degree between the fusion event and the other event of the candidate coincidence event and the target event corresponding to the target detector module. In response to the matching degree being greater than or equal to a preset matching degree threshold, the second processing card 630 may determine whether each of the plurality of single events corresponding to the fusion event and the other event meets the preset condition.

The matching degree refers to an evaluation parameter used to measure a degree of consistency between the fusion event and the other event in feature dimensions such as time and energy, and is used to characterize whether the fusion event and the other event have a possibility of forming a coincidence event pair. In some embodiments, the second processing card 630 may perform a predictive matching evaluation based on information of the fusion event and features of the other event without expanding the plurality of single events contained in the fusion event. For example, the second processing card 630 may convert the information into vector data and calculate a similarity as the matching degree.

The preset matching degree threshold refers to a matching degree threshold used to distinguish between "possibly coincident" and "obviously non-coincident," and is configured to control whether a single event enters subsequent fine coincidence selection. The preset matching degree threshold may be set based on experience or requirements. It is understandable that after the predictive matching based on the matching degree, the second processing card 630 may further perform coincidence selection based on the plurality of single events corresponding to the fusion event and the other event, to determine whether a coincidence condition is satisfied and which specific single event satisfies the coincidence condition.

In some embodiments, the matching degree may be related to at least one of a count, a time span, or an energy span of the plurality of single events corresponding to the fusion event.

The count of the plurality of single events corresponding to the fusion event refers to the count of single events that constitute the fusion event and is configured to reflect an event density level within the fusion event. The time span refers to a distribution range of the plurality of single events corresponding to the fusion event in the time dimension. For example, it may be the difference between the occurrence time of the earliest single event and the occurrence time of the latest single event. The energy span refers to a distribution range of the plurality of single events corresponding to the fusion event in the energy dimension. For example, it may be the difference between the maximum energy and the minimum energy.

In some embodiments, the matching degree decreases accordingly when the count is large, or when the time span or the energy span is large. Specific values may be determined based on a preset table, a preset algorithm, or the like.

By introducing the matching degree for coincidence determination, coarse-grained matching judgment may first be performed based on fusion event, and then fine coincidence determination may be performed by tracing back to single events in the fusion event when necessary. This reduces ineffective expansion of single events in the fusion event and improves the overall efficiency of coincidence determination.

In some embodiments, after obtaining the coincidence event pairs, the second processing card 630 may perform image reconstruction based on the coincidence event pairs to obtain a PET image. In some embodiments, the second processing card 630 is further configured to send control instructions to the detector rings to control the normal operation of the multiple detector rings. For example, the second processing card 630 may send a recovery instruction and a timing instruction to the multiple detector rings. The second processing card 630 may also supply power to the multiple detector rings to ensure the normal operation of the multiple detector rings.

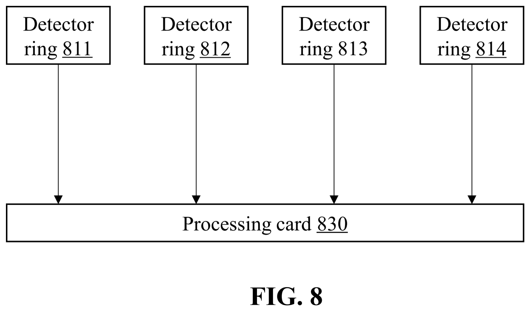

In some embodiments, two or more components in the PET system 600 may be combined into a component, and the component may implement functions of the two or more components. For example, the first processing card 620 and the second processing card 630 may be combined into a component (e.g., a processing card). The component may be configured to determine target events based on detection data, process the target events, and determine coincidence event pairs. FIG. 8 is a schematic diagram illustrating an exemplary structure of a PET system according to some embodiments of the present disclosure. As illustrated in FIG. 8, the embodiments of the present disclosure provide a PET system, which includes multiple detector rings 811-814 and a processing card 830. In some embodiments, functions corresponding to the processing card 830 may be executed by the processing device 120.

The processing card 830 may be regarded as an integrated card combining the first processing card 620 and the second processing card 630. In some embodiments, the processing card 830 is communicatively connected to the multiple detector rings and is configured to receive detection data from the multiple detector rings, determine a target event based on the target single event within the same time granularity, detected by detector modules at the same circumferential position across the multiple detector rings, and determine the coincidence event pair by processing the target event.

According to some embodiments of the present disclosure, the PET system includes the multiple detector rings, at least one data processing card and at least one coincidence event card. Each of the multiple detector rings includes a plurality of detector modules and has an axial index respectively. The data processing card is communicatively connected to the multiple detector rings, wherein the data processing card is configured to receive detection data with different axial indexes from the multiple detector rings, and determine a target event based on the detection data. The coincidence event card is configured to determine a coincidence event pair by processing the target event, wherein the coincidence event pair at least includes coincidence events detected by the multiple detector rings.

In some embodiments, an axial dimension of the each of the multiple detector rings is greater than or equal to 15 cm, a length of an axial field of view of the PET system is greater than or equal to 60 cm.

In some embodiments, the coincidence event pair is determined by detection data received by detector modules respectively belonging to two adjacent detector rings, or coincidence event pair is determined by detection data received by detector modules respectively belonging to two non-adjacent detector rings.

According to some embodiments of the present disclosure, the PET system includes the multiple detector rings, the plurality of first processing cards, and the second processing card. The multiple detector rings are arranged side by side along the axial direction of the PET system, and each of the multiple detector ring includes a plurality of detector modules arranged along a circumferential direction of the multiple detector rings. Each first processing card is communicatively connected to the multiple detector rings and is configured to receive the detection data detected by the plurality of detector modules at the same circumferential position across the multiple detector rings, and determine the target event based on the detection data. The second processing card is communicatively connected to the plurality of first processing cards and configured to determine the coincidence event pair by processing the target event. Thus, a count of redundant events in the axial direction of the multiple detector rings can be compressed before coincidence determination, reducing the data scale and the computational complexity of the coincidence determination.

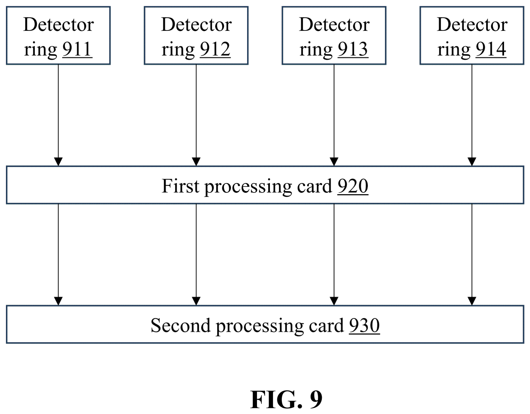

FIG. 9 is a schematic diagram illustrating an exemplary structure of a PET system according to some embodiments of the present disclosure. As illustrated in FIG. 9, the embodiments of the present disclosure provide a PET system, which includes multiple detector rings 911-914, a first processing card 920, and a second processing card 930. In some embodiments, the PET system may include only one first processing card 920. More descriptions of the multiple detector rings, the first processing card, and the second processing card may be found elsewhere in the present disclosure.

In some embodiments, the first processing card is further configured to group the detection data. The grouping refers to dividing the detection data according to a preset grouping rule to obtain data subsets. The grouping rule may be set based on experience or requirements. For example, the grouping rule may be determined based on circumferential positions, opposite relationships, module index ranges of detector modules corresponding to the detection data, or a combination thereof. Merely by way of example, each of the multiple detector rings has four detector modules A-D. The first processing card may group detector modules A and B on the multiple detector rings into one group and group detector modules C and D on the multiple detector rings into another group. As another example, the first processing card may group detector module A on the multiple detector rings into one group, group detector module B on the multiple detector rings into another group, group detector module C on the multiple detector rings into another group, and group detector module D on the multiple detector rings into another group.

In some embodiments, the first processing card may include a plurality of first processing sub-cards. A first processing sub-card refers to one of a plurality of coincidence event sub-cards split from the first processing card. Each first processing sub-card may be an independent hardware module or a logically partitioned unit on the same hardware carrier.

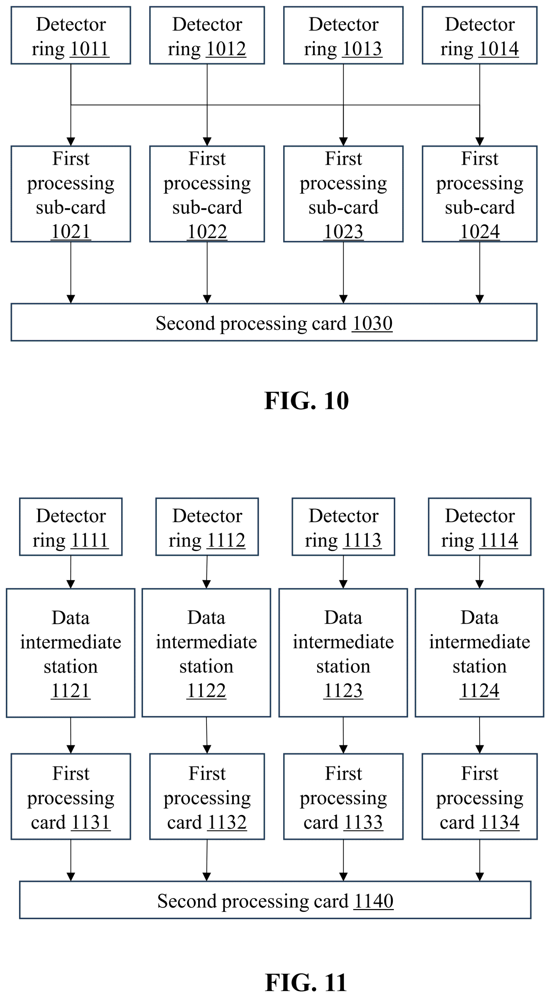

FIG. 10 is a schematic diagram illustrating an exemplary structure of a PET system according to some embodiments of the present disclosure. As illustrated in FIG. 10, the embodiments of the present disclosure provide a PET system, which includes multiple detector rings 1011-1014, a plurality of first processing sub-cards 1021-1024, and a second processing card 1030.

Each first processing sub-card is configured to determine the target event based on each grouped detection data. It is understandable that when the first processing card needs to process massive single event data, the first processing card may be decomposed into the plurality of first processing sub-cards to work collaboratively. Each first processing sub-card independently handles a data subset assigned to it. For example, the first processing card may be divided into a first processing sub-card 1 and a first processing sub-card 2. The first processing sub-card 1 may be configured to process detection data detected by the group of detector modules A and B. The first processing sub-card 2 may be configured to process detection data detected by the group of detector modules C and D. As another example, as illustrated in FIG. 10, the first processing sub-card 1021 may be configured to process detection data detected by the group of detector modules A on the multiple detector rings 1011-1014. The first processing sub-card 1022 may be configured to process detection data detected by the group of detector modules B on the multiple detector rings 1011-1014. The first processing sub-card 1023 may be configured to process detection data detected by the group of detector modules C on the multiple detector rings 1011-1014. The first processing sub-card 1024 may be configured to process detection data detected by the group of detector modules D on the multiple detector rings 1011-1014. Using the plurality of first processing sub-cards for grouped processing can improve the efficiency of target event determination.

In some embodiments, the PET system may include a cable bundle covering cables between the first processing sub-card and the detector module corresponding to each grouped detection data. The embodiment does not limit the material and size of the cable bundle, as long as it can implement its function.

In some embodiments, the second processing card may be further configured to group the target event. The grouping refers to dividing the target events according to a preset grouping rule to obtain event subsets. The grouping rule may be set based on experience or requirements. For example, the grouping rule may be determined based on circumferential positions, opposite relationships, module index ranges of detector modules corresponding to the target event, or a combination thereof. Merely by way of example, each detector ring has four detector modules A-D. Detector modules A of the multiple detector rings is at a same circumferential position across the multiple detector rings, detector modules B of the multiple detector rings is at a same circumferential position across the multiple detector rings, detector modules C of the multiple detector rings is at a same circumferential position across the multiple detector rings, detector modules D of the multiple detector rings is at a same circumferential position across the multiple detector rings. The detector modules A and detector modules C of the multiple detector rings are opposing modules. The detector modules B and detector modules D of the multiple detector rings are opposing modules. The second processing card may group target events corresponding to detector modules A and C on the multiple detector rings into one group, group target events corresponding to detector modules B and D on the multiple detector rings into another group. The two groups are respectively processed by two different second processing sub-cards.

In some embodiments, the second processing card may include a plurality of second processing sub-cards, each second processing sub-card being configured to process each grouped target event. The second processing sub-card refers to one of the plurality of coincidence event sub-cards split from the second processing card. Each second processing sub-card may be an independent hardware module or a logically partitioned unit on the same hardware carrier. It is understandable that each second processing sub-card being configured to process each grouped target event means that the second processing sub-card processes only its corresponding group of target events, without having to process all target events. Data processing between different second processing sub-cards is independent of each other. By providing the plurality of second processing sub-cards, the processing load can be shared, and the processing capability of the PET system under high data volume can be improved.

In some embodiments, a switching card is provided between the plurality of first processing sub-cards and the plurality of second processing sub-cards, the switching card is configured to dynamically route target events determined by the plurality of first processing sub-cards. The switching card refers to a data exchange and scheduling unit provided between the plurality of first processing sub-cards and the plurality of second processing sub-cards, and is configured to manage a transmission path of the target events. The switching card may exist as an independent hardware card or as a dedicated switching module in the second processing card. The dynamic routing refers to dynamically selecting a transmission path for the target events among the plurality of first processing sub-cards and the plurality of second processing sub-cards according to an operating state of the PET system, rather than adopting a fixed one-to-one correspondence. That is, an output of a first processing sub-card is not simply and fixedly directed to a specific second processing sub-card. Instead, according to a real-time operating state of the PET system, the most suitable second processing sub-card can be dynamically selected for each batch of data.

In some embodiments, the switching card may route the target events based on at least one of a current processing load of each second processing sub-card, an operating state or a fault state of each second processing sub-card, or the availability of a data transmission link. For example, a target event determined by a first processing sub-card A is normally sent to a second processing sub-card A. However, when the second processing sub-card A is overloaded or fails, the target event can be sent to a second processing sub-card B instead.

By introducing a schedulable data distribution layer between target event determination and coincidence event pair determination, the two-level processing units are logically decoupled, and data processing continuity is maintained when the loads of the plurality of second processing sub-cards are uneven or when a second processing sub-card fails.

In some embodiments, the second processing card further includes a scheduling card. The scheduling card refers to a control and decision unit provided in the second processing card and is configured to sense and/or schedule an overall operating state of target event determination and coincidence event pair determination. The scheduling card does not directly participate in the determination of the target events or the coincidence event pairs, but is configured to generate a control strategy. In some embodiments, the scheduling card is configured to guide actions of the first processing card and the second processing card based on the detection data and operation information of the first processing card and the second processing card. The operation information refers to information related to operating states of the first processing card and the second processing card. In some embodiments, the operation information may include at least one of a processing load, a data throughput, a cache occupancy, or an abnormal or fault state. In some embodiments, guiding the actions of the first processing card and the second processing card may include guiding the time granularity used for determining the target events, guiding the route used for determining the target events, guiding control of whether the first processing card or the second processing card participates in a current processing flow, or the like.

In some embodiments, the scheduling card may cooperate with the switching card. For example, a routing strategy, guided by the scheduling card, for determining the target events may be sent to the switching card. The switching card then forwards the target events according to the routing strategy.

By introducing the scheduling card, dynamic scheduling of target event determination and coincidence event pair determination can be achieved, and the adaptability of the PET system to load changes can be improved.

In some embodiments, the PET system further includes a preprocessing card, communicatively connected to the plurality of first processing cards (or the first processing card) and the second processing card, and is configured to preprocess the detection data. The preprocessing card may be provided between the multiple detector rings and the first processing card(s), or between the first processing card(s) and the second processing card. In some embodiments, the preprocessing card is configured to preprocess the detection data. The preprocessing may include at least one of screening out single events whose energy does not satisfy a preset energy condition, marking abnormal events or invalid events, or preliminarily grouping or sorting the detection data.

By providing the preprocessing card, invalid single events entering subsequent target event determination and coincidence event pair determination stages can be reduced, and a processing load can be lowered.

In some embodiments, the PET system further includes a data intermediate station communicatively connected to the multiple detector rings and the first processing card(s), and configured to transmit data from the multiple detector rings to the first processing card(s) actively or passively. FIG. 11 is a schematic diagram illustrating an exemplary structure of a PET system according to some embodiments of the present disclosure. As illustrated in FIG. 11, the PET system includes multiple detector rings 1111-1114, data intermediate stations 1121-1124, a plurality of first processing cards 1131-1134, and a second processing card 1140. The data intermediate stations 1121-1124 are communicatively connected to the multiple detector rings1111-1114 and the first processing cards 1131-1134, and are integrated with a PET frame or disposed close to the PET frame.

In some embodiments, the data intermediate station may include a data interface or a data relay point.

The data intermediate stations 1121-1124 may be configured to transmit data collected by the multiple detector rings 1111-1114 to the plurality of first processing cards 1131-1134 actively or passively. The active refers to that the data intermediate stations 1121-1124 provide energy for the detection data of the detector rings 1111-1114 during transmission. The passive refers to the data intermediate stations 1121-1124 directly transmitting data of the detector rings 1111-1114 to the first processing cards 1131-1134.

In some embodiments, the detector rings 1111-1114 are connected to the data intermediate stations 1121-1124 via optical fibers. The data of the detector rings 1111-1114 are optical signals. After providing energy for the transmitted optical signals, the data intermediate stations 1121-1124 transmit the optical signals to the first processing cards 1131-1134.

From a physical implementation perspective, transmitting data of the multiple detector rings to a same downstream processing unit (e.g., the first processing card(s), the first processing sub-card, or the like) requires long cable connections. Long-distance transmission may cause signal attenuation. By providing the data intermediate station, on one hand, energy may be provided for the transmitted optical signals; on the other hand, subsequent expansion and upgrade of the PET system may be facilitated, i.e., facilitating an increase in the count of detector rings in the PET system. It is understandable that the data intermediate station has access points. New detector rings can be directly connected via the access points. If the data intermediate station is not provided, excessively long cables would need to be routed from the detector rings to the downstream processing unit, affecting signal transmission.

In some embodiments, the PET system further includes a storage module connected to the second processing card and configured to store the coincidence event pairs. The storage module and the second processing card may be connected via a wired connection or wirelessly. This embodiment does not limit this. The storage module may be a memory. This embodiment does not limit a type or structure of the storage module, as long as its function can be achieved.

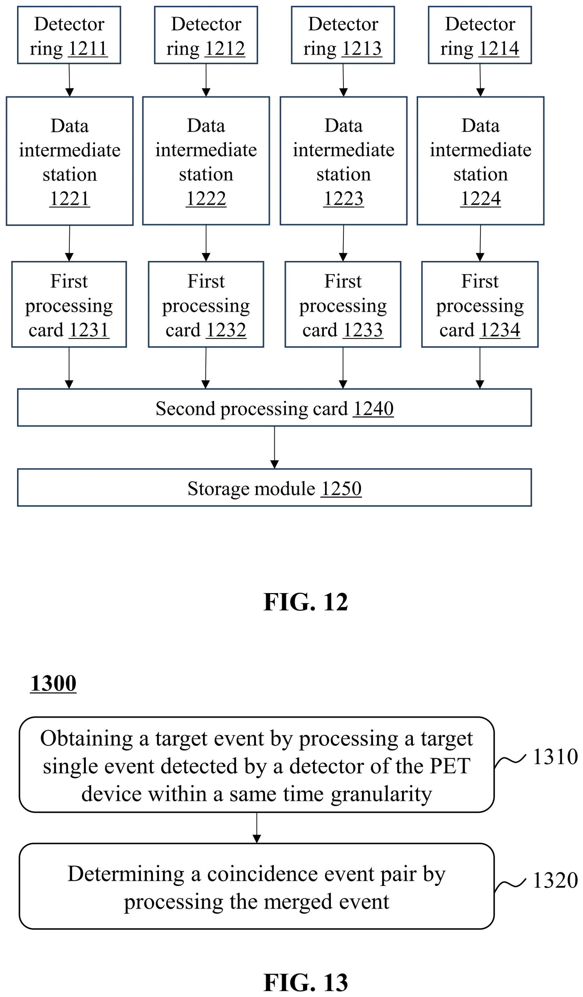

FIG. 12 is a schematic diagram illustrating an exemplary structure of a PET system according to some embodiments of the present disclosure. As illustrated in FIG. 12, the PET system includes multiple detector rings 1211-1214, data intermediate stations 1221-1224, a plurality of first processing cards 1231-1234, a second processing card 1240, and a storage module 1250.

After determining the coincidence event pairs, the second processing card 1240 may transmit the coincidence event pairs to the storage module 1250 for storage. By providing the storage module, the coincidence event pairs processed by the second processing card can be stored, facilitating subsequent processing.

FIG. 13 is a flowchart illustrating an exemplary process for performing coincidence determination according to some embodiments of the present disclosure. In some embodiments, a process 1300 may be performed by the processing device 120. As illustrated in FIG. 13, the process 1300 may include the steps 1310-1320.

In 1310, determining a target event based on a target single event, detected by detector modules at a same circumferential position across the multiple detector rings of the PET imaging device.

In some embodiments, the operation 1310 may be performed by a first processing card(s) (e.g., the first processing card 620, the first processing card 920, the first processing sub-cards 1021-1024, the first processing sub-cards 1131-1134, or the first processing card 1231-1234). More descriptions of obtaining the target event may be found elsewhere in the present disclosure (e.g., FIG. 6 and the description thereof).

In 1320, determining a coincidence event pair by processing the target event. In some embodiments, the operation 1320 may be performed by a second processing card (e.g., the second processing card 630, the second processing card 930, the second processing card 1030, the second processing card 1140, or the second processing card 1240). More descriptions of determining the coincidence event pair may be found elsewhere in the present disclosure (e.g., FIG. 6 and the description thereof).

In some embodiments, the 1310 and the 1320 are performed by a processing card (e.g., the processing card 830).

FIG. 14 is a schematic diagram illustrating an exemplary structure of a chip according to some embodiments of the present disclosure. As illustrated in FIG. 14, the chip 1400 includes a processor 1410. In some embodiments, the processing device 120, the first processing card (e.g., the first processing card 620, the first processing card 920, the first processing sub-cards 1021-1024, the first processing sub-cards 1131-1134, or the first processing card 1231-1234), and/or the second processing card (e.g., the second processing card 630, the second processing card 930, the second processing card 1030, the second processing card 1140, or the second processing card 1240) may be implemented on the chip 1400.

In some embodiments, as illustrated in FIG. 14, the chip 1400 further includes a memory 1420. The processor 1410 may invoke and run a computer program from the memory 1420 to implement one or more functions described in some embodiments of the present disclosure. In some embodiments, the memory 1420 may be a separate device independent of the processor 1410, or the memory 1420 may be integrated in the processor 1410.

In some embodiments, as illustrated in FIG. 14, the chip 1400 further includes an input interface 1430. The processor 1410 may control the input interface 1430 to communicate with other devices or chips; specifically, the processor 1410 may obtain information or data sent by the other devices or chips.

In some embodiments, as illustrated in FIG. 14, the chip 1400 further includes an output interface 1440. The processor 1410 may control the output interface 1440 to communicate with other devices or chips, specifically, the processor 1410 may output information or data to the other devices or chips.

It is understandable that the chip described in the embodiments of the present disclosure may also be referred to as a system-on-chip, a system chip, a chip system, or a system-on-a-chip or the like.

One or more embodiments of the present disclosure further provide a computer device, an internal structure diagram of which is illustrated in FIG. 15. FIG. 15 is a schematic diagram illustrating an exemplary internal structure of a computer device according to some embodiments of the present disclosure. In some embodiments, the processing device 120 may be implemented on the computer device illustrated in FIG. 15. The computer device includes a processor, a memory, a communication interface, a display screen, and an input device connected through a system bus. The processor of the computer device is configured to provide computing and control capabilities. The memory of the computer device includes a non-volatile storage medium and an internal memory. The non-volatile storage medium stores the operating system and a computer program. The internal memory provides an environment for executing the operating system and the computer program stored in the non-volatile storage medium. The communication interface of the computer device is configured to communicate with an external terminal via a wired or wireless manner, and the wireless manner may be implemented through Wi-Fi, a mobile cellular network, Near Field Communication (NFC), or other technologies. The computer program, when executed by the processor, implements a method implemented one or more functions described in some embodiments of the present disclosure. The display screen of the computer device may be a liquid crystal display screen or an electronic ink display screen; the input device of the computer device may be a touch layer overlaying the display screen, or may be a key, a trackball, or a touchpad provided on a housing of the computer device, or may also be an external keyboard, a touchpad, or a mouse or the like.

It is understandable by those skilled in the art that the structure illustrated in FIG. 15 is merely a block diagram illustrating a part of the structure relevant to the embodiments of the present disclosure, and does not constitute a limitation on the computer device to which the embodiments of the present disclosure are applied. A specific computer device may include more or fewer components than those illustrated in the figure, may combine some components, or may have a different component arrangement.

One or more embodiments of the present disclosure provide a computer device, including a memory and a processor, where the memory stores a computer program, and the processor, when executing the computer program, implements one or more functions described in some embodiments of the present disclosure.

One or more embodiments of the present disclosure provide a computer program product, including a computer program, where the computer program, when executed by a processor, implements one or more functions described in some embodiments of the present disclosure.

One or more embodiments of the present disclosure provide a non-transitory computer readable medium, comprising executable instructions that, when executed by at least one processor, direct the at least one processor to perform one or more functions described in some embodiments of the present disclosure.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of this disclosure.

Moreover, certain terminology has been used to describe embodiments of the present disclosure. For example, the terms "one embodiment," "an embodiment," and/or "some embodiments" may mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.), or combining software and hardware implementation that may all generally be referred to herein as a "unit," "module," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB. NET, Python, or the like, conventional procedural programming languages, such as the "C" programming language, Visual Basic, Fortran 2103, Perl, COBOL 2102, PHP, ABAP, dynamic programming languages such as Python, Ruby, and Groovy, or other programming languages. The program code may execute entirely on the user's computer, partly on the user's computer, as a stand-alone software package, partly on the user's computer, and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations, therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, for example, an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed object matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±1%, ±5%, ±10%, or ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. A PET system, comprising:
multiple detector rings arranged side by side along an axial direction of the PET system, each of the multiple detector rings including a plurality of detector modules arranged along a circumferential direction of the detector ring;
a first processing card communicatively connected to the multiple detector rings, wherein the first processing card is configured to receive detection data with a same circumferential index, and determine a target event based on the detection data; and
a second processing card communicatively connected to the first processing card, and configured to determine a coincidence event pair by processing the target event.

2. The PET system of claim 1, wherein to determine the target event, the first processing card is further configured to:
determine the target event based on a target single event within a same time granularity in the detection data.

3. The PET system of claim 2, wherein to determine the target event, the first processing card is further configured to:
in response to the target single event including one single event, designate the single event as the target event; and
in response to the target single event including at least two single events, select one from the at least two single events as the target event.

4. The PET system of claim 3, wherein to select the one from the at least two single events as the target event, the first processing card is further configured to:
select, randomly or based on a preset rule, the one from the at least two single events as the target event.

5. The PET system of claim 1, wherein to determine the coincidence event pair, the second processing card is further configured to:
determine a candidate coincidence event based on the target event;
determine a target event that meets a preset condition from target events, the target events being within a preset time window and corresponding to target detector modules, the target detector modules being positioned opposite or approximately opposite to the detector module corresponding to the candidate coincidence event;
designate the candidate coincidence event and the target event that meets the preset condition as a candidate coincidence event pair; and
determine the coincidence event pair based on the candidate coincidence event pair.

6. The PET system of claim 1, wherein
the first processing card is further configured to group the detection data; and
the first processing card includes a plurality of first processing sub-cards, each of the plurality of first processing sub-cards being configured to determine the target event based on each grouped detection data.

7. The PET system of claim 6, wherein the PET system further includes a cable bundle covering cables between the first processing sub-card and detector modules corresponding to the each grouped detection data.

8. The PET system of claim 6, wherein
the second processing card is further configured to group the target event; and
the second processing card includes a plurality of second processing sub-cards, each of the plurality of second processing sub-cards being configured to process each grouped target event.

9. The PET system of claim 8, wherein a switching card is provided between the plurality of first processing sub-cards and the plurality of second processing sub-cards, the switching card being configured to dynamically route target events determined by the plurality of first processing sub-cards.

10. The PET system of claim 1, wherein the second processing card further includes a scheduling card configured to guide actions of the first processing card and the second processing card based on the detection data and operation information of the first processing card and the second processing card.

11. The PET system of claim 1, wherein the PET system further includes a preprocessing card communicatively connected to the first processing card and the second processing card, and configured to preprocess the detection data.

12. The PET system of claim 1, wherein the PET system further includes a data intermediate station communicatively connected to the multiple detector rings and the plurality of first processing cards, and configured to transmit data from the multiple detector rings to the plurality of first processing cards actively or passively.

13. The PET system of claim 12, wherein the data intermediate station includes a data interface or a data relay point.

14. The PET system of claim 1, wherein the PET system further includes a storage module connected to the second processing card and configured to store the coincidence event pair.

15. The PET system of claim 1, wherein the PET system further includes a frame, and the multiple detector rings are sequentially arranged along an axial direction of the frame.
